(19) **Europäisches Patentamt / European Patent Office / Office européen des brevets**

(11) **EP 4 157 222 B1**

(12) **FASCICULE DE BREVET EUROPEEN**

(45) Date de publication et mention de la délivrance du brevet:
**26.06.2024 Bulletin 2024/26**

(21) Numéro de dépôt: **21726923.2**

(22) Date de dépôt: **20.05.2021**

(51) Classification Internationale des Brevets (IPC):
**A61K 9/107** (2006.01)    **A61K 47/10** (2017.01)
**A61K 47/14** (2017.01)    **A61K 47/44** (2017.01)
**A61K 47/20** (2006.01)

(52) Classification Coopérative des Brevets (CPC):
**A61K 9/1075; A61K 9/0009; A61K 47/10;**
**A61K 47/14; A61K 47/20; A61K 47/44**

(86) Numéro de dépôt international:
**PCT/EP2021/063495**

(87) Numéro de publication internationale:
**WO 2021/239578 (02.12.2021 Gazette 2021/48)**

(54) **EMULSIONS POUR LIBERATION CONTROLEE DE MEDICAMENTS**

**EMULSIONEN ZUR KONTROLLIERTEN WIRKSTOFFFREISETZUNG**

**EMULSIONS FOR CONTROLLED DRUG RELEASE**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **27.05.2020 FR 2005611**

(43) Date de publication de la demande:
**05.04.2023 Bulletin 2023/14**

(73) Titulaires:
 • **Avignon Universite**
  **84029 Avignon Cedex 1 (FR)**
 • **CENTRE NATIONAL DE LA RECHERCHE
  SCIENTIFIQUE**
  **75794 Paris Cedex 16 (FR)**
 • **SORBONNE UNIVERSITE**
  **75006 Paris (FR)**
 • **Institut National de la Santé et de la Recherche
  Médicale**
  **75013 Paris (FR)**
 • **Université de Montpellier**
  **34090 Montpellier (FR)**
 • **Ecole Nationale Supérieure de Chimie de
  Montpellier**
  **34296 Montpellier Cedex 5 (FR)**

(72) Inventeurs:
 • **AL RIFAI, Nour**
  **92160 ANTONY (FR)**
 • **CONTINO-PEPIN, Christiane**
  **84210 ALTHEN DES PALUDS (FR)**
 • **DESGRANGES, Stéphane**
  **84000 AVIGNON (FR)**
 • **TAULIER, Nicolas**
  **75014 PARIS (FR)**

(74) Mandataire: **Osha BWB**
  **2, rue de la Paix**
  **75002 Paris (FR)**

(56) Documents cités:
  **WO-A1-2016/185425    WO-A2-03/000295**

 • **WOOSTER T.J. ET AL.: "Impact of Oil Type on
  Nanoemulsion Formation and Ostwald Ripening
  Stability", LANGMUIR, vol. 24, no. 22, 18
  novembre 2008 (2008-11-18), pages 12758-12765,
  XP055769906, US ISSN: 0743-7463, DOI:
  10.1021/la801685v**

**Description**

**Domaine technique de l'invention**

La présente description concerne une émulsion pour une utilisation thérapeutique, une émulsion, et des formulations sèches obtenues par lyophilisation desdites émulsions.

**Etat de la technique**

La libération de médicaments dans un organisme vivant peut entraîner l'apparition d'effets secondaires. La sélection d'une concentration en médicament résulte souvent d'un compromis entre seuil thérapeutique et toxicité. Ce compromis peut se révéler insatisfaisant.

Il est connu que la libération de médicaments déclenchée par un stimulus externe (lumière, champ magnétique, ultrasons, rayonnement micro-ondes) peut être adaptée pour un traitement thérapeutique local ciblé, incluant un contrôle spatial, temporel et/ou de dosage du médicament libéré.

Dans ce contexte, la libération de médicaments déclenchée par ultrasons (« Ultrasound-triggered drug delivery ») est une méthode émergente. Le mécanisme de libération implique généralement la cavitation de bulles, la vaporisation d'un composé perfluorocarboné présent dans la phase discontinue d'une émulsion, e.g., des gouttelettes, et/ou une élévation importante de température.

Ainsi, par exemple, le document EP 3 095 806 A1 décrit des nanoémulsions de gouttelettes utilisées comme vecteurs d'un médicament ou d'une substance thérapeutique, comprenant des composés perfluorocarbonés (PFC) stabilisées par des dendrimères amphiphiles appelés H/F-DendriTAC, et leurs utilisations en tant qu'outils théranostiques pour la détection et le traitement de cancers. Cependant, ce type de vecteur peut être d'application limitée dans la mesure où un composé PFC est utilisé en tant que composant principal des gouttelettes, dans lequel de nombreuses substances thérapeutiques, notamment les substances lipophiles, sont insolubles. Le rôle du composé PFC liquide dans ces nanoémulsions est de déclencher la libération de médicament du fait de sa vaporisation lorsque la nanoémulsion est soumise à un champ acoustique de pression suffisante. Le document EP 3 095 806 A1 néglige l'exigence (et ne décrit pas une façon) de prévenir la libération passive du médicament contenu dans les gouttelettes lors de l'utilisation thérapeutique des nanoémulsions.

En outre, l'article de T. Wooster et al., Impact of Oil Type on Nanoemulsion Formation and Ostwald Ripening Stability, Langmuir (2008), 24, pp. 12758-12765, s'intéresse à l'impact du type d'huile contenue dans des nanoémulsions sur la stabilité de l'émulsion au mûrissement d'Ostwald (ou OR, pour « Ostwald Ripening »). En vue d'augmenter une telle résistance à l'OR, l'article suggère d'utiliser des nanoémulsions dont la phase huileuse contient au moins 50% d'un triglycéride insoluble dans l'eau. L'article demeure totalement silencieux sur l'encapsulation ou la libération de médicaments par de telles nanoémulsions.

La demande de brevet WO 03/000295A2 décrit des compositions comprenant des « véhicules lipophiles » permettant d'améliorer la biodisponibilité d'inhibiteurs de la CETP. Cette demande néglige la possibilité (et ne suggère en rien) de déclencher une libération contrôlée de ces inhibiteurs au moyen d'un stimulus externe.

**Résumé de l'invention**

Dans la présente description et dans les revendications, le terme « comprendre » signifie la même chose que « inclure », « contenir », et est inclusif ou ouvert et n'exclut pas d'autres éléments non décrits ou représentés. En outre, dans la présente description, les termes « environ » et « substantiellement » sont synonymes de (signifient la même chose que) une marge inférieure et/ou supérieure de 10%, par exemple 5%, de la valeur respective.

Un des objectifs de la présente description est de fournir une émulsion capable de libérer une substance incorporée dans une phase discontinue de l'émulsion, et notamment utilisable dans un cadre thérapeutique en tant que vecteur de médicament. Un des objectifs de la présente description est de fournir une émulsion capable d'éviter et/ou de limiter les effets secondaires du médicament incorporé dans la phase discontinue de l'émulsion sur, par exemple, l'organisme d'un patient cible de la thérapie. Par effets secondaires, on entend des effets indésirables que le médicament peut provoquer, et que l'on souhaite éviter dans le cadre de l'utilisation thérapeutique de l'émulsion. Par exemple, afin d'éviter les phénomènes de résistance des bactéries, de hautes doses d'antibiotiques sont fréquemment administrées, menant à des effets secondaires importants.

Selon un premier aspect, la présente description concerne une émulsion pour une utilisation thérapeutique par libération contrôlée de médicament déclenchée par application d'un stimulus externe. L'émulsion comprend une phase discontinue incluant des gouttelettes et une phase aqueuse continue. Les gouttelettes comprennent un premier et un second composé lipophile, au moins un tensioactif et au moins un médicament. Le premier composé lipophile, qui peut former un mélange avec le second composé lipophile, a une solubilité massique dans l'eau supérieure à la solubilité massique dans l'eau du second composé lipophile. Le ratio volumique du premier composé lipophile au second composé lipophile est supérieur à 70 : 30. La phase discontinue est substantiellement exempte ou complètement dépourvue de composé perfluorocarboné.

Les inventeurs ont constaté avec surprise que la coprésence, dans la phase discontinue, d'un premier et d'un second composé lipophile ayant des solubilités massiques dans l'eau distinctes à la même température, dans ledit ratio volumique du premier composé lipophile au second composé lipophile, à savoir un ratio volumique supérieur à 70 : 30, permet de conférer plusieurs propriétés inattendues à l'émulsion dans le cadre de son utilisation thérapeutique par libération contrôlée de médicament. En comparaison d'une émulsion où les gouttelettes ne comprendraient qu'un seul composé lipophile, le diamètre moyen D des gouttelettes de l'émulsion peut par exemple être réduit par la présence du second composé lipophile. La réduction du diamètre conférée par la présence du second composé lipophile permet de rendre les gouttelettes à la fois résistantes à une déstabilisation physique par séparation gravitationnelle, floculation et/ou coalescence. De tels phénomènes de déstabilisation physique des gouttelettes peuvent engendrer la libération du médicament sans qu'un stimulus externe ne soit nécessaire pour la provoquer. Cette libération passive ou retardée du médicament s'oppose à la libération contrôlée que l'on peut obtenir dans le cadre de l'utilisation thérapeutique de l'émulsion telle que définie dans le premier aspect de la présente description. La présence du second composé lipophile à solubilité distincte dans ledit ratio volumique permet de prévenir cette fuite du médicament hors des gouttelettes et ainsi de déclencher la libération du médicament à un moment prédéterminé, par exemple lorsque les gouttelettes de l'émulsion atteignent une localisation cible de la thérapie. La réduction du diamètre des gouttelettes permet en outre de rendre les gouttelettes modulables selon les exigences de tailles de gouttelettes pouvant être requises par une utilisation thérapeutique spécifique afin d'améliorer l'efficacité de la thérapie, par exemple, afin de bénéficier de l'effet EPR (pour « Enhanced Permeability and Rétention ») dans un tissu cancéreux.

En outre, la présence dudit second composé lipophile dans la phase discontinue dans ledit ratio volumique supérieur à 70 : 30 permet de stabiliser les gouttelettes vis-à-vis du mûrissement d'Ostwald. Le mûrissement d'Ostwald est un phénomène conduisant à la déstabilisation des gouttelettes d'une émulsion, en entraînant la croissance des gouttelettes les plus grosses d'une émulsion au détriment des plus petites, dont la taille diminue. Ce phénomène de déstabilisation est notamment favorisé par l'inhomogénéité d'une émulsion, *i.e.* lorsque l'émulsion présente un indice de polydispersité PDI élevé, et par la solubilité dans la phase continue, *i.e.* la phase aqueuse dans le cas des émulsions telles que définies dans la présente description, des composés compris dans les petites gouttelettes, qui ont tendance à « fuir » les petites gouttelettes en diffusant hors de celles-ci. Les inventeurs ont constaté qu'un ratio volumique du premier composé lipophile au second composé lipophile supérieur à 70 : 30 était suffisant pour prévenir le mûrissement d'Ostwald de façon satisfaisante dans le cadre de l'utilisation thérapeutique des émulsions par libération contrôlée de médicament.

Dans la présente description et dans les revendications, par « émulsion », on comprend une dispersion de la phase discontinue dans la phase aqueuse continue. Des caractéristiques d'une émulsion, comme par exemple la valeur du diamètre moyen des gouttelettes D, ou encore l'indice de polydispersité PDI, peuvent évoluer avec le temps. Cependant, dans la présente description et dans les revendications, par « émulsion », on entend l'émulsion sous une forme utilisable en thérapie, *e.g.*, sous une forme administrable dans un organisme vivant. Par exemple, l'émulsion selon la présente description correspond à l'émulsion prête à l'administration de l'émulsion. Par exemple, lorsque l'émulsion est destinée à être administrée par injection intraveineuse dans l'organisme d'un patient, l'émulsion selon la présente description correspond à l'émulsion quelques secondes avant injection intraveineuse de l'émulsion dans l'organisme du patient. Selon une ou plusieurs formes de réalisation, l'émulsion administrable peut correspondre à l'émulsion obtenue au moment de sa préparation.

Lors de l'utilisation thérapeutique de l'émulsion telle que définie dans le premier aspect de la présente description, la libération du médicament n'est pas retardée ou passive, mais peut être contrôlée par application d'un stimulus externe. La libération du médicament est contrôlée en cela qu'elle est induite ou déclenchée à un moment prédéterminé, par exemple lorsque les gouttelettes de l'émulsion atteignent une localisation cible de la thérapie.

L'utilisation thérapeutique comprend la libération de médicament, par exemple la libération de médicament dans un organisme humain ou animal.

La libération du médicament peut être déclenchée par application d'un stimulus externe, comme par exemple des ultrasons, par application d'un champ ultrasonore. Selon une ou plusieurs formes de réalisation, le stimulus externe est choisi parmi le groupe comprenant la lumière, un champ magnétique, les ultrasons, les microondes. Selon une ou plusieurs formes de réalisation de l'émulsion pour une utilisation thérapeutique par libération contrôlée de médicament, la libération du médicament est déclenchée par des ultrasons. Dans ces formes de réalisation, la libération contrôlée de médicament peut ainsi être localisée à la focale d'un faisceau d'ondes ultrasonores, ce dernier pouvant être dirigé, par exemple, sur un tissu ou organe à traiter, *i.e.,* un tissu ou organe cible de la thérapie.

Selon une ou plusieurs formes de réalisation, l'utilisation thérapeutique de l'émulsion comprend l'administration de l'émulsion dans un organisme vivant. Selon une ou plusieurs formes de réalisation, l'administration de l'émulsion à l'organisme vivant se fait par voie intraveineuse, voie orale, voie topique, voie intranasale, voie pulmonaire, voie trans-muqueuse, ou combinaisons de celles-ci. Selon une ou plusieurs formes de réalisation, l'administration de l'émulsion se fait par voie intraveineuse.

L'effet thérapeutique recherché dans le cadre de l'utilisation de l'émulsion peut varier selon la nature du médicament inclus dans les gouttelettes de l'émulsion. De façon générale, selon une ou plusieurs formes de réalisation, le médicament présente une activité biologique ayant un impact bénéfique sur la santé humaine. Selon une ou plusieurs formes de réalisation, le médicament est un médicament anticancéreux, antiinflammatoire, antioxydant, antithrombotique, antibactérien, antiviral, antifongique, antiparasitaire, ou une combinaison de ceux-ci.

Selon une ou plusieurs formes de réalisation, le médicament est un antibiotique, par exemple un antibiotique sélectionné dans le groupe comprenant les fluoroquinones, les macrolides, les lincosamides, les tigécyclines.

Selon une ou plusieurs formes de réalisation, les gouttelettes de l'émulsion comprennent en outre des ligands spécifiques de récepteurs de l'organisme dans lequel l'émulsion est administrée. De cette façon, le contrôle du transport du médicament dans l'organisme et/ou le ciblage de tissus ou organes d'intérêt comme par exemple des tumeurs, des sites d'infection ou d'inflammation, peut être amélioré. Selon une ou plusieurs formes de réalisation, les ligands sont hydrophiles. De tels ligands hydrophiles peuvent se placer, substantiellement, au niveau du tensioactif encapsulant le « coeur » de la gouttelette, à savoir au niveau de la « coquille » de la gouttelette, *i.e.,* au niveau de l'interface avec la phase aqueuse continue.

Selon une ou plusieurs formes de réalisation, la première solubilité massique dans l'eau à 25 °C est au moins 10 fois plus grande que la seconde solubilité massique dans l'eau à 25 °C, par exemple 20 fois plus grande, 50 fois plus grande, 70 fois plus grande, 100 fois plus grande.

Selon une ou plusieurs formes de réalisation, la première solubilité massique dans l'eau à 25 °C est d'au moins 0,1 $\mu$g/mL, par exemple comprise entre 0,1 $\mu$g/mL et 1 g/mL, par exemple entre 0,1 $\mu$g/mL et 100 mg/mL, entre 0,1 $\mu$g/mL et 1 mg/mL, entre 0,1 $\mu$g/mL et 100 $\mu$g/mL, entre 1 $\mu$g/mL et 20 $\mu$g/mL, ou encore entre 0,1 mg/mL et 100 mg/mL. Selon une ou plusieurs formes de réalisation, la seconde solubilité massique dans l'eau à 25 °C est inférieure à 1 $\mu$g/mL, par exemple inférieure à 0,1 $\mu$g/mL, inférieure à 0,01 $\mu$g/mL. Afin d'évaluer la solubilité de composés lipophiles solides à température ambiante (*e.g.*, environ 20°C) ayant des solubilités inférieures à 100 $\mu$g/mL, une méthode consiste à liquéfier ces composés en augmentant la température au-delà de 60°C, pour en déposer 80 mg sur une feuille d'aluminium de 2.5 cm$^2$. Après refroidissement à 25°C, le composé lipophile forme une fine couche solide sur la feuille d'aluminium. Cette feuille est alors trempée pendant au moins 48 h dans un bécher contenant 500 mL d'eau en constante agitation grâce à un barreau magnétique. La différence de poids (mesurée avec une balance à haute précision) de la feuille sur laquelle se trouve le composé lipophile avant et après son trempage donne alors la quantité de composé lipophile qui a pu se solubiliser dans l'eau. Pour les composés lipophiles liquides à température ambiante (*e.g.*, environ 20°C), ayant des solubilités inférieures à 100 $\mu$g/mL, la solubilité peut par exemple être mesurée par HPLC couplée à une détection spectroscopique, *e.g.*, UV, visible, et/ou IR. Pour des solubilités supérieures à 100 $\mu$g/mL, la solubilité du composé lipophile peut par exemple être mesurée par des méthodes classiques de mesures spectroscopique en analysant la variation de l'absorbance en fonction de la concentration croissante du composé lipophile en solution dans l'eau.

Selon une ou plusieurs formes de réalisation, le mélange de composés lipophiles est présent dans un pourcentage en poids par rapport au poids total de la phase discontinue supérieur à 60 % pds, par exemple supérieur à 70 % pds, supérieur à 80 % pds, supérieur à 90 % pds.

Selon une ou plusieurs formes de réalisation, l'émulsion est une nanoémulsion, c'est-à-dire que la phase discontinue comprend des gouttelettes de taille nanométrique, *i.e.* de diamètre moyen D inférieur à 1 $\mu$m. Dans la présente description et dans les revendications, sauf indication du contraire, on entend par diamètre moyen D des gouttelettes le diamètre moyen des gouttelettes tel que déterminé par la théorie de Mie et mesuré en utilisant un instrument de diffusion dynamique de la lumière, comme décrit dans la description détaillée. Dans la présente description et dans les revendications, sauf indication du contraire, par indice de polydispersité (ou PDI) d'une émulsion, on entend le second moment normalisé dans l'ajustement par la méthode dite des cumulants, comme décrit dans la description détaillée.

Sauf indication du contraire, dans la présente description et dans les revendications le diamètre moyen D et l'indice de polydispersité PDI auxquels on se réfère correspondent aux caractéristiques de l'émulsion sous une forme administrable, telle que définie plus haut et, par exemple, correspondant à l'émulsion quelques secondes avant l'injection de l'émulsion dans un organisme, lorsque l'émulsion est destinée à être administrée sous cette forme.

Selon une ou plusieurs formes de réalisation, les gouttelettes ont un diamètre moyen D compris entre 50 nm et 900 nm. En dessous de 50 nm, la capacité de charge des gouttelettes en médicament peut être trop faible pour certaines utilisations thérapeutiques. Selon une ou plusieurs formes de réalisation, les gouttelettes ont un diamètre moyen D compris entre 100 nm et 600 nm. De telles plages de diamètre moyen D peuvent par exemple permettre de cibler spécifiquement les tumeurs cancéreuses grâce à l'effet EPR (pour "Enhanced Permeability and Rétention"). Les particules de cette taille ont en effet tendance à davantage s'accumuler au niveau des cellules tumorales que dans les tissus sains. Cela se produit en raison de plusieurs phénomènes biologiques dont les deux plus importants sont le développement anormal des vaisseaux sanguins et l'inefficacité du drainage lymphatique dans les tissus cancéreux.

Selon une ou plusieurs formes de réalisation, les gouttelettes ont un diamètre moyen D compris entre 50 nm et 200 nm, par exemple entre 50 nm et 100 nm, ou entre 100 nm et 200 nm. Une plage de diamètre moyen D comprise entre 50 nm et 100 nm peut par exemple permettre aux gouttelettes de franchir la barrière hémato-encéphalique et atteindre le cerveau si ce dernier est ciblé par la thérapie. Selon une ou plusieurs formes de réalisation, l'indice de polydispersité PDI de l'émulsion est inférieur à 0,4, par exemple inférieur à 0,25.

Comme mentionné plus haut, les inventeurs ont constaté qu'afin d'obtenir des émulsions utilisables pour une utilisation thérapeutique par libération contrôlée de médicament, le ratio volumique du premier composé lipophile au second composé lipophile devait être supérieur à 70 : 30. Selon une ou plusieurs formes de réalisation, le ratio volumique du premier composé lipophile au second composé lipophile est supérieur à 90 : 10, par exemple supérieur à 95 : 5, et par exemple compris entre 95 : 5 et 99 : 1. Les inventeurs ont constaté avec surprise que la présence du second composé dans le mélange de composés lipophiles dans de tels ratios permettait de stabiliser l'émulsion et de prévenir la libération passive ou retardée de médicaments selon une ou plusieurs formes de réalisation de l'utilisation thérapeutique de l'émulsion selon le premier aspect.

Selon une ou plusieurs formes de réalisation, le mélange de composés lipophiles et l'au moins un tensioactif sont biocompatibles. Par substance biocompatible, on fait référence à une substance sans effets toxiques ou nuisibles sur la santé et utilisable pour des applications pharmaceutiques. Par exemple, selon une ou plusieurs formes de réalisations, le mélange de composés lipophiles et l'au moins un tensioactif peuvent être considérés inoffensifs selon les critères reconnus par l'USFDA (United States Food and Drug Administration) et bénéficier de l'appellation « GRAS » (pour « Generally Recognized As Safe »).

Selon une ou plusieurs formes de réalisation, le second composé lipophile comprend au moins une chaîne alkyle linéaire d'au moins 6 atomes de carbone, par exemple d'au moins 8 atomes de carbone. Selon une ou plusieurs formes de réalisation, le second composé lipophile comprend au moins une chaîne alkyle linéaire en C8 à C70, par exemple en C20 à C60. Selon une ou plusieurs formes de réalisation, le second composé lipophile comprend au moins une chaîne alkyle linéaire en C8 à C20, par exemple en C8 à C10, par exemple en C10 à C18.

Selon une ou plusieurs formes de réalisation, le mélange de composés lipophiles est substantiellement exempt ou complètement dépourvu de composé perfluorocarboné (ou composé PFC). En tout état de cause, selon le premier aspect de la présente description, la phase discontinue est substantiellement exempte ou complètement dépourvue de composé PFC. Dans des applications thérapeutiques en combinaison avec la libération de médicaments déclenchée par ultrasons (« Ultrasound-triggered drug delivery »), plusieurs médicaments d'intérêt peuvent être solubles dans une telle phase discontinue. De telles émulsions offrent donc une capacité de charge en médicament améliorée (« drug-loading capacity ») par rapport aux émulsions dans lesquelles la phase discontinue est substantiellement constituée de composé PFC. Selon une ou plusieurs formes de réalisation, le mélange de composés lipophiles comprend substantiellement des composés lipidiques. Un tel mélange de composés lipophiles permet d'augmenter la capacité de charge en médicament des gouttelettes. Selon une ou plusieurs formes de réalisation, le premier composé lipophile est un composé lipidique sélectionné dans le groupe comprenant les mono-, di- ou tri-esters de glycérol, ou dérivés du glycérol, les mono-, di- ou tri- ou tétra-esters de l'acide citrique ou dérivés de l'acide citrique, les acides gras, les monoesters d'acides gras, les stérides, les sphingolipides, les glycérophospholipides, les polycétides, les saccharolipides, les terpènes, les lipides dérivés du prénol, et combinaisons de ceux-ci.

Par exemple, selon une ou plusieurs formes de réalisation, le premier composé lipophile comprend ou consiste en un mono- ou un di-ester d'un dérivé du glycérol. Par exemple, lorsque le dérivé du glycérol comprend ou consiste en du propylène glycol, le premier composé lipophile peut comprendre ou consister en un mono-, un dicaprylate de propylène glycol ou un mélange de ceux-ci.

Selon une ou plusieurs formes de réalisation, combinables avec les formes de réalisation précédentes, le second composé lipophile est un composé lipidique sélectionné dans le groupe comprenant les mono-, di- ou triesters de glycérol, ou dérivés du glycérol, les mono-, di- ou tri- ou tétra-esters de l'acide citrique ou dérivés de l'acide citrique, les acides gras, les monoesters d'acides gras, les stérides, les sphingolipides, les glycérophospholipides, les polycétides, les saccharolipides, les terpènes, les lipides dérivés du prénol, les huiles essentielles, les substituts de graisse, les cires, et combinaisons de ceux-ci.

Lors de l'utilisation thérapeutique de telles émulsions contenant les composés lipidiques susnommés selon une ou plusieurs formes de réalisation, sans vouloir être liés par une quelconque théorie, les inventeurs pensent que l'application d'un stimulus externe, *e.g.*, les ultrasons, pour déclencher la libération contrôlée du médicament, a pour effet d'augmenter la perméabilité de la « coquille » des gouttelettes, à savoir la partie des gouttelettes se situant substantiellement à la surface des gouttelettes, c'est-à-dire au niveau de l'interface avec la phase aqueuse continue. Cette augmentation de la perméabilité peut par exemple être provoquée par une force de radiation acoustique lorsque les gouttelettes sont soumises à des ultrasons, et elle facilite la diffusion du médicament hors des gouttelettes le temps de l'application du stimulus externe. Pour certaines utilisations thérapeutiques, il est également possible d'observer une synergie de l'action du stimulus externe et du médicament dans l'effet thérapeutique obtenu en utilisant les émulsions.

Selon une ou plusieurs formes de réalisation, la libération contrôlée de médicament n'est pas déclenchée par une cavitation et/ou une vaporisation d'un composé présent dans les gouttelettes, mais par augmentation de la perméabilité des gouttelettes. Selon une ou plusieurs formes de réalisation, l'utilisation selon le premier aspect n'implique pas de changement d'état du mélange de composés lipophiles et/ou la thérapie ne s'accompagne pas d'élévation importante de température qui pourrait conduire à des effets secondaires, tels que par exemple la brûlure du tissu cible de la thérapie.

Selon une ou plusieurs formes de réalisation, la concentration volumique de la phase discontinue dans la phase aqueuse continue est comprise entre 0,001 % v/v et 90 % v/v, et la concentration du médicament en poids/volume par rapport au volume total du mélange de composés lipophiles dans l'émulsion est comprise entre 10 $\mu$g/mL et 100 mg/mL. Selon une ou plusieurs formes de réalisation, la concentration volumique de la phase discontinue dans la phase aqueuse continue est comprise entre 0,000001 % v/v et 90 % v/v, par exemple entre 0,00001 % v/v et 10 % v/v, entre 0,0001 et 1% v/v, ou encore entre 0,10 % v/v et 2 % v/v. Selon une ou plusieurs formes de réalisation, la concentration du médicament en poids/volume par rapport au volume total du mélange de composés lipophiles dans l'émulsion est comprise entre 10 $\mu$g/mL et 10 mg/mL.

Selon une ou plusieurs formes de réalisation, l'au moins un tensioactif est sélectionné dans le groupe comprenant les dendrimères de type Dendri-TAC, les oligomères de type $F_iTAC_n$ ou $H_iTAC_n$, le TPGS 1000, le TPGS 750M, les tensioactifs dérivés d'acide-aminés et/ou dérivés de sucres tels que les polyglycosides d'alkyle, et combinaisons de ceux-ci.

Selon une ou plusieurs formes de réalisation, l'au moins un tensioactif est un oligomère de type $F_iTAC_n$ ou $H_iTAC_n$. De tels composés confèrent non seulement une stabilité améliorée aux gouttelettes, mais présentent également une bonne biocompatibilité. Ils comprennent une partie hydrophile (ou « tête polaire ») comprenant un oligomère de type polyTRIS (pour « poly Tris(hydroxyméthyl)aminométhane »), et une partie hydrophobe comprenant une chaîne linéaire alkyle, laquelle est fluorée pour les oligomère de type $F_iTAC_n$. Le nombre $n$ est le degré d'oligomérisation de la partie polyTRIS et $i$ est le nombre d'atomes de carbone de la chaîne linéaire alkyle.

Selon une ou plusieurs formes de réalisation, i est compris entre 5 et 20, par exemple entre 10 et 15 et $n$ est compris entre 5 et 20, par exemple entre 5 et 10.

Selon une ou plusieurs formes de réalisation, le composé amphiphile comprend un dendrimère de type Dendri-TAC, tel que ceux décrits dans la demande de brevet EP3095806 A1. Les Dendri-TAC constituent une nouvelle classe de composés amphiphiles biocompatibles possédant des propriétés d'auto-assemblage et hautement modulables, que ce soit au niveau de la queue hydrophobe ou de la multiplication des branches de la tête hydrophile. Les tensioactifs de type Dentri-TAC offrent de nombreuses opportunités pour l'encapsulation de médicaments, du fait de leur architecture moléculaire dendritique permettant l'apparition de cavités internes pouvant constituer autant de compartiments dans lesquels le médicament peut être stocké avant libération.

Selon une ou plusieurs formes de réalisation, l'émulsion comprend, dans la phase aqueuse et/ou la phase discontinue, un excipient sélectionné dans le groupe comprenant les agents acidifiants, les agents alcalinisants, les agents tampons, les stabilisants comme par exemple les cryoprotecteurs, les lyoprotecteurs. Selon une ou plusieurs formes de réalisation, l'excipient est présent substantiellement dans la phase aqueuse de l'émulsion.

Selon une ou plusieurs formes de réalisation, l'émulsion comprend :

- une phase discontinue comprenant des gouttelettes incluant un mélange de composés lipophiles, au moins un tensioactif et au moins une substance thérapeutique la phase discontinue étant substantiellement exempte ou complètement dépourvue de composé perfluorocarboné ;
- une phase aqueuse continue ;

où :

- le mélange de composés lipophiles comprend un premier composé lipophile ayant une première solubilité massique dans l'eau et un second composé lipophile ayant une seconde solubilité massique dans l'eau inférieure à la première solubilité massique, dans un ratio massique du premier composé lipophile au second composé lipophile supérieur à 70 : 30 ;

où le mélange de composés lipophiles est présent dans un pourcentage en poids par rapport au poids total de la phase discontinue supérieur à 60 % pds ; et
où :

- le premier composé lipophile est sélectionné dans le groupe comprenant la triacétine, la tripropionine, la tributyrine, l'acétyltributylcitrate, et combinaisons de ceux-ci ;
- le second composé lipophile est sélectionné dans le groupe comprenant les triglycérides en C8 à C10, les triglycérides en C10 à C18, et combinaisons de ceux-ci.

Selon un deuxième aspect, la présente description concerne une formulation sèche pour une utilisation thérapeutique par libération contrôlée de médicament déclenchée par application d'un stimulus externe, ladite formulation étant obtenue par lyophilisation d'une émulsion telle que celle définie dans le premier aspect. La formulation sèche est donc substantiellement exempte d'eau.

L'utilisation thérapeutique de la formulation sèche par libération contrôlée de médicament déclenchée par application d'un stimulus externe peut comprendre l'administration de la formulation sèche dans un organisme cible de la thérapie. Selon une ou plusieurs formes de réalisation, l'administration de la formulation sèche dans l'organisme cible de la thérapie est effectuée par voie topique, par voir orale, par voie intranasale ou combinaisons de celles-ci. Selon une ou plusieurs formes de réalisation, la formulation sèche permet également un stockage à long terme avant une utilisation sous forme liquide, *e.g.*, pour une administration par voie intraveineuse, après addition d'eau ou d'une solution aqueuse à la formulation sèche. Selon un autre aspect ne faisant pas partie de l'objet revendiqué, la présente description concerne une méthode thérapeutique, comprenant les étapes suivantes :

- l'administration d'une émulsion telle que définie dans le premier aspect de la présente description ainsi que dans l'une quelconque des formes de réalisation décrites *vide supra,* ou d'une formulation sèche telle que définie dans le deuxième aspect la présente description et dans ses différentes formes de réalisation, dans un organisme ; et
- l'application d'un stimulus externe à un moment prédéterminé pour déclencher la libération contrôlée du médicament.

Selon une ou plusieurs formes de réalisation, le stimulus externe comprend des ultrasons. Selon une ou plusieurs formes de réalisation, le moment prédéterminé correspond au moment auquel les gouttelettes provenant de l'émulsion ou de la formulation sèche ont atteint une localisation cible de l'organisme. Selon une ou plusieurs formes de réalisation, l'administration comprend l'injection de l'émulsion par voie intraveineuse dans l'organisme.

Des formes de réalisation selon les aspects référencés ci-dessus ainsi que des avantages supplémentaires apparaîtront à la lecture de la description détaillée suivante et des revendications annexées.

## Brève description des figures

D'autres avantages et caractéristiques de l'invention apparaîtront à la lecture de la description, illustrée par les figures suivantes :

[Fig. 1], représente un schéma illustrant une forme de réalisation d'émulsion pour une utilisation thérapeutique, par libération contrôlée de médicament ;

[Fig. 2], représente un schéma illustrant un exemple comparatif d'émulsion différente des émulsions selon la présente description, donnant lieu à une libération passive de médicament.

[Fig. 3], représente un histogramme illustrant une forme de réalisation d'une émulsion pour une utilisation thérapeutique, par libération contrôlée de médicament, dans différentes conditions d'application d'ultrasons.

## Description détaillée

Dans la description détaillée suivante des formes de réalisation de la présente description, de nombreux détails spécifiques sont exposés afin de fournir une compréhension plus approfondie de la présente description. Cependant, il apparaîtra à l'homme du métier que la présente description peut être mise en oeuvre sans ces détails spécifiques. Dans d'autres cas, des caractéristiques bien connues n'ont pas été décrites en détail pour éviter de compliquer inutilement la description.

La présente description fournit des exemples non limitants d'émulsions pour une utilisation thérapeutique par libération contrôlée de médicament, et fournit également des détails non limitants concernant la préparation des émulsions ainsi que leurs utilisations thérapeutiques potentielles.

I. Illustration de l'intérêt du second composé lipophile dans le cadre d'utilisations thérapeutiques de l'émulsion

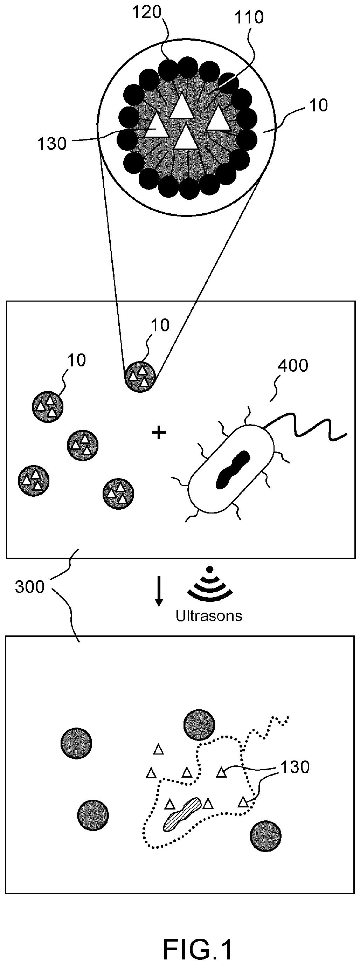

La Figure 1 illustre un exemple d'émulsion pour une utilisation thérapeutique par libération contrôlée de médicament selon la présente description. L'utilisation illustrée par la Figure 1 comprend l'administration de ladite émulsion dans un organisme, en vue de cibler un organe 300 compris dans ledit organisme et infecté par des bactéries 400. L'émulsion pour une utilisation thérapeutique selon la présente description peut également cibler d'autres organismes nuisibles. Dans d'autres formes de réalisation selon la présente description, la thérapie peut par exemple cibler des virus, cellules tumorales, champignons, parasites, et combinaisons de ceux-ci.

Comme le montre la Figure 1, les gouttelettes 10 provenant de l'émulsion incluent un premier et un second composé lipophile formant un mélange 110, un tensioactif 120 et un médicament 130. Le mélange de composés lipophiles 110 comprend un premier composé lipophile et un second composé lipophile ayant une solubilité massique dans l'eau inférieure à celle du premier composé lipophile, dans un ratio volumique du premier composé lipophile au second composé lipophile supérieur à 70 : 30. La présence d'un tel second composé lipophile dans le mélange 110 permet de stabiliser les gouttelettes 10 lors de l'utilisation thérapeutique de l'émulsion. Le mélange lipophile est en outre présent, dans la forme de réalisation décrite ici, dans un pourcentage en poids par rapport au poids total de la phase discontinue supérieur à 60 % pds.

Comme le montre la Figure 1, dans cette forme de réalisation, un stimulus externe est appliqué sous forme d'ultrasons pour déclencher la libération contrôlée du médicament 130. Le stimulus externe est appliqué à un moment prédéterminé, correspondant dans cet exemple au moment où les gouttelettes atteignent le voisinage de la bactérie 400. Cependant, selon une ou plusieurs formes de réalisation d'émulsions pour une utilisation thérapeutique selon la présente description, d'autres stimuli externes peuvent être envisagés, *e.g.*, des ondes lumineuses, électromagnétiques, ainsi que d'autres moments prédéterminés, *e.g.*, un moment où une certaine concentration de gouttelettes est détectée au voisinage de la cible de la thérapie.

Dans cette forme de réalisation, la libération du médicament 130 est contrôlée dans la mesure où la libération est déclenchée au voisinage de la bactérie 400, et non pas au voisinage, par exemple, d'un tissu sain. Comme le montre la Figure 1, la libération contrôlée du médicament 130 conduit à la destruction ciblée de la bactérie 400.

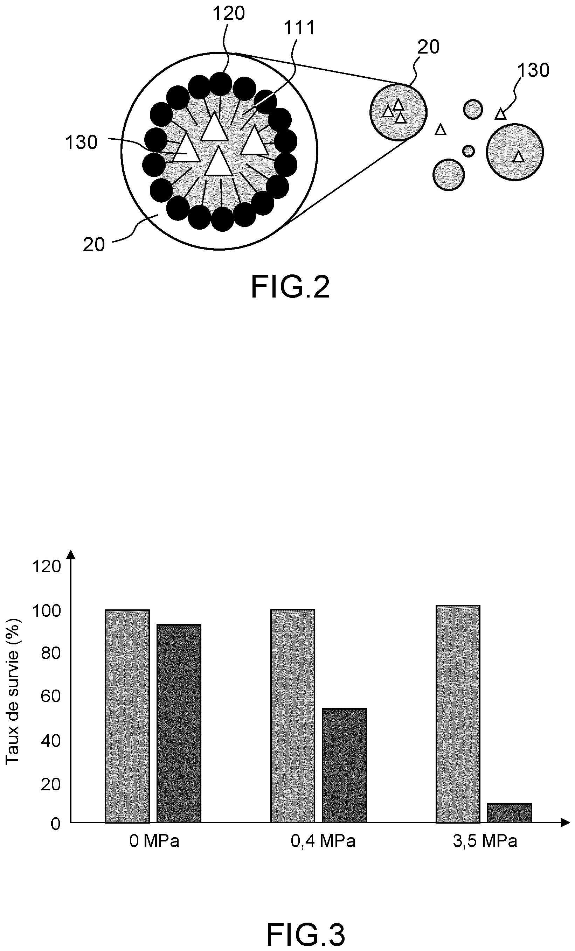

La Figure 2 illustre un exemple comparatif d'émulsion donnant lieu à une libération passive de médicament. Dans cet exemple comparatif, les gouttelettes 20 proviennent d'une émulsion différente des émulsions de la présente description, en cela qu'elles incluent un unique composé lipophile 111, au lieu de la coprésence de composés lipophiles tel que défini plus haut, notamment d'un mélange comprenant un premier composé lipophile ayant une première solubilité massique dans l'eau et un second composé lipophile ayant une seconde solubilité massique dans l'eau inférieure à la première solubilité massique, dans un ratio volumique du premier composé lipophile au second composé lipophile supérieur à 70 : 30.

Comme le montre la Figure 2, la taille des gouttelettes 2 est moins uniforme, et leur stabilité est largement inférieure aux gouttelettes de l'émulsion illustrée dans la Figure 1.

II. Exemple d'émulsion pour une utilisation thérapeutique par libération contrôlée de médicament

*Préparation de l'émulsion 1:*

Dans cet exemple de préparation d'émulsion référencée émulsion 1 ci-après, la lévofloxacine, le méthanol, l'acétonitrile et l'acétyltributylcitrate (ATBC), ont été commandés chez Sigma-Aldrich (Saint-Quentin Fallavier, France). La Suppocire® A a été commandée chez Gattefosse, et Esherichia coli BW25113 de type sauvage chez Coli Genetic Stock Center. Dans cet exemple, les gouttelettes incluent $H_{12}TAC_7$ en tant que tensioactif, pouvant par exemple être synthétisé par polymérisation radicalaire, en une étape, selon un protocole décrit dans la littérature [Contino-Pepin et al., 2002, « Amphiphilic Oligomers: A New Kind of Macromolecular Carrier of Antimitotic Drugs », Curr. Med. Chem. - Anti-Cancer Agents, 2, 645-665].

Environ 11 mM de $H_{12}TAC_7$ sont dissouts dans 15 mL dans tube à centrifuger en plastique contenant 4 mL d'eau contenant 9 ‰ de NaCl, pour former une solution micellaire. Le premier composé lipophile, ATBC (0,57 mL), et le second composé lipophile, Suppocire® A (0,03 mL), sont alors ajoutés, *i.e.,* dans un ratio volumique de ATBC à la Suppocire® A de 95 : 5. Le médicament, la lévofloxacine, un antibiotique de la famille des fluoroquinolones, est préalablement solubilisé dans l'ATBC, à une concentration de 1,77 mg/mL. Il est donc présent à une concentration en poids/volume d'environ 1,68 mg/mL par rapport au volume total du mélange de composés lipophiles dans l'émulsion. Le mélange obtenu est ensuite mélangé en utilisant un vortex pour former une émulsion grossière. L'émulsion grossière est ensuite émulsifiée en utilisant un homogénéiseur haute pression (modèle LV1 de Microfluidics) : 8 passages sont effectués à une pression de 138 MPa. L'émulsion résultante est ensuite centrifugée à une force relative centrifuge de 2000 g pendant 30 s, avec une mini-centrifugeuse de table (Mini Star de VWR). Le surnageant est séparé et centrifugé cette fois à 17000 g pendant 1 h en utilisant une centrifugeuse de table (micro Star 17R de VWR) de sorte que les gouttelettes forment un comprimé. Le surnageant est alors retiré et remplacé par une solution fraîche de NaCl 9 ‰. L'émulsion obtenue peut être stockée au réfrigérateur à 4°C pendant 3 jours. L'émulsion a été diluée 1000 fois pour obtenir l'émulsion 1, dont l'utilisation thérapeutique par libération contrôlée de médicament selon le deuxième aspect est décrite *vide infra.*

*Mesure du diamètre moyen des gouttelettes :*

Le diamètre moyen des gouttelettes est mesuré par diffusion dynamique de la lumière (ou DLS, pour « Dynamic Light Scattering »). Un appareil comprenant un goniomètre basé sur la plate-forme ALV/CGS-3 a été utilisé. L'émulsion soumise à la mesure est typiquement diluée 100 fois et la mesure est effectuée à 20°C à différents angles de diffusion :

de 50° à 130° avec un palier de 20°. A chaque angle θ, l'appareil fournit un taux de décroissance dont la valeur est tracée en fonction de l'amplitude du vecteur de diffusion q(θ) exprimée selon l'équation (1) suivante :

**[Math 1]**

$$q(\theta) = \frac{4\pi n}{\lambda} \sin(\theta/2)$$

où n = 1,333 est l'indice de réfraction de la solution et λ est la longueur d'onde du laser. Un ajustement de la courbe selon la méthode des cumulant fournit le diamètre moyen D, ainsi que l'indice de polydispersité PDI.

L'émulsion 1 dont la préparation est décrite *vide supra* présente un diamètre moyen D de gouttelettes 248 ± 11 nm et un indice de polydispersité PDI de 0,24. A titre de comparaison, une émulsion par ailleurs identique mais ne comprenant pas de médicament, une émulsion dite « blanche », présente un diamètre moyen D de 240 ± 9 nm et un indice de polydispersité PDI de 0,12. En outre, une émulsion blanche par ailleurs identique mais ne comprenant pas de second composé lipophile tel que défini dans la présente description en référence aux premiers et seconds aspects, présente un diamètre moyen D bien plus élevé, de 868 ± 40 nm et un indice de polydispersité PDI de 0,15.

*Utilisation thérapeutique de l'émulsion 7, par libération contrôlée de médicament :*

Dans cet exemple d'utilisation thérapeutique de l'émulsion 1 dont la préparation est décrite *vide supra,* l'effet thérapeutique de l'émulsion 1, contenant de la lévofloxacine en tant que médicament, a été testé envers des souches sauvages de bactéries *Escherichia coli* (*E. Coli*). Dans cet exemple, des ultrasons ont été utilisés en tant que stimulus externe permettant la libération contrôlée de lévofloxacine dans des tissus modèles.

Les résultats de l'exposition de tissus modèles contenant des bactéries et également chargés en émulsion 1 sont présentés dans la Figure 3 et détaillés *vide infra.* Trois différentes conditions expérimentales sont illustrées, selon la pression pic-à-pic des ondes ultrasonores appliquées aux tissus modèles. Les trois conditions expérimentales sont par ailleurs identiques et comprennent 20 mn d'irradiation ultrasonore (rapport cyclique de 5%, fréquence de répétition de pulsation de 200 Hz) à une température de 37°C. Après incubation et/ou application d'ultrasons dans les tissus modèles, les bactéries ont été dénombrées selon des protocoles connus de l'homme du métier (non détaillés).

Comme le montre la Figure 3, en l'absence d'ultrasons, *i.e.*, à une pression pic-à-pic de 0 MPa, la présence de l'émulsion 1 dans le tissu modèle le taux de survie de bactéries décroît de 100% (en l'absence d'émulsion et d'ultrasons) à 91,7%, ce qui correspond à une libération passive de médicament très réduite en l'absence de stimulus externe. Comme le montre également la Figure 3, le taux de survie de bactéries diminue drastiquement pour atteindre des valeurs de 53% et de 8,5%, à des valeurs de pressions pic-à-pic, respectivement, de 0,4 MPa et 3,5 MPa.

Des expériences de contrôle ont consisté à tester, dans les mêmes conditions, l'émulsion blanche correspondante à l'émulsion 1, *i.e.*, une émulsion dépourvue de lévofloxacine mais par ailleurs identique à l'émulsion 1 (expériences comparatives correspondant aux barres grisées sur la Figure 3). La Figure 3 montre qu'une telle émulsion blanche ne montre substantiellement aucun effet thérapeutique, car le taux de survie des bactéries reste pratiquement inchangé dans les trois conditions testées (100%, 100% et 102%, à des valeurs de pressions pic-à-pic, respectivement, de 0 MPa, 0,4 MPa et 3,5 MPa).

Bien que décrits à travers un certain nombre d'exemples de réalisation, les aspects selon la présente description comprennent différentes variantes, modifications et perfectionnements qui apparaîtront de façon évidente à l'homme de l'art, étant entendu que ces différentes variantes, modifications et perfectionnements font partie de la portée de l'invention telle que définie par les revendications qui suivent.

**Revendications**

1. Emulsion pour une utilisation thérapeutique par libération contrôlée de médicament déclenchée par application d'un stimulus externe, comprenant :

   - une phase discontinue comprenant des gouttelettes incluant un premier et un second composé lipophile, au moins un tensioactif et au moins un médicament; et
   - une phase aqueuse continue ;

   dans laquelle le premier composé lipophile a une première solubilité massique dans l'eau et le second composé lipophile a une seconde solubilité massique dans l'eau inférieure à la première solubilité massique, dans un ratio volumique du premier composé lipophile au second composé lipophile supérieur à 70 : 30 ; et dans laquelle la phase discontinue est substantiellement exempte ou complètement dépourvue de composé perfluorocarboné.

2. Emulsion pour une utilisation thérapeutique selon la revendication 1, dans laquelle la première solubilité massique dans l'eau à 25 °C est au moins 10 fois plus grande que la seconde solubilité massique dans l'eau à 25 °C.

3. Emulsion pour une utilisation thérapeutique selon l'une quelconque des revendications précédentes, dans laquelle :

   - la première solubilité massique dans l'eau à 25 °C est d'au moins 0,1 $\mu$g/mL, et/ou
   - la seconde solubilité massique dans l'eau à 25 °C est inférieure à 1 $\mu$g/mL.

4. Emulsion pour une utilisation thérapeutique selon l'une quelconque des revendications précédentes, dans laquelle le premier et le second composé lipophile forment un mélange présent dans un pourcentage en poids par rapport au poids total de la phase discontinue supérieur à 60 % pds.

5. Emulsion pour une utilisation thérapeutique selon l'une quelconque des revendications précédentes, dans laquelle les gouttelettes ont un diamètre moyen D compris entre 50 nm et 900 nm et l'indice de polydispersité PDI est inférieur à 0,4.

6. Emulsion pour une utilisation thérapeutique selon l'une quelconque des revendications précédentes, dans laquelle le ratio volumique du premier composé lipophile au second composé lipophile est compris entre 95 : 5 et 99 : 1.

7. Emulsion pour une utilisation thérapeutique selon l'une quelconque des revendications précédentes, dans laquelle le second composé lipophile comprend au moins une chaîne alkyle linéaire d'au moins 6 atomes de carbone.

8. Emulsion pour une utilisation thérapeutique selon l'une quelconque des revendications 1-6, dans laquelle :

   - le premier composé lipophile est sélectionné dans le groupe comprenant les mono-, di- ou tri-esters de glycérol, ou dérivés du glycérol, les mono-, di-, tri- ou tétra-esters de l'acide citrique ou dérivés de l'acide citrique, les acides gras, les monoesters d'acides gras, les stérides, les sphingolipides, les glycérophospholipides, les polycétides, les saccharolipides, les terpènes, les lipides dérivés du prénol, et combinaisons de ceux-ci ; et/ou
   - le second composé lipophile est sélectionné dans le groupe comprenant les mono-, di- ou triesters de glycérol, ou dérivés du glycérol, les mono-, di-, tri- ou tétra-esters de l'acide citrique ou dérivés de l'acide citrique, les acides gras, les monoesters d'acides gras, les stérides, les sphingolipides, les glycérophospholipides, les polycétides, les saccharolipides, les terpènes, les lipides dérivés du prénol, les huiles essentielles, les substituts de graisse, les cires, et combinaisons de ceux-ci.

9. Emulsion pour une utilisation thérapeutique selon l'une quelconque des revendications 1-6, dans laquelle :

   - le premier composé lipophile est sélectionné dans le groupe comprenant l'acétyltributylcitrate, un mono- ou un dicaprylate de propylène glycol, et combinaisons de ceux-ci ; et/ou
   - le second composé lipophile est sélectionné dans le groupe comprenant des triglycérides en C8 à C10, des triglycérides en C10 à C18, et combinaisons de ceux-ci.

10. Emulsion pour une utilisation thérapeutique selon l'une quelconque des revendications précédentes, dans laquelle la concentration volumique de la phase discontinue dans la phase aqueuse continue est comprise entre 0,000001 % v/v et 90 % v/v, et/ou dans laquelle la concentration du médicament en poids/volume par rapport au volume total du mélange de composés lipophiles dans l'émulsion est comprise entre 10 $\mu$g/mL et 100 mg/mL.

11. Emulsion pour une utilisation thérapeutique selon l'une quelconque des revendications précédentes, dans laquelle l'au moins un tensioactif est sélectionné dans le groupe comprenant les dendrimères de type Dendri-TAC, les oligomères de type $F_iTAC_n$ ou $H_iTAC_n$, le TPGS 1000, le TPGS 750M, les tensioactifs dérivés de sucres et/ou d'acide-aminés, et combinaisons de ceux-ci.

12. Formulation sèche pour une utilisation thérapeutique par libération contrôlée de médicament déclenchée par application d'un stimulus externe, ladite formulation sèche étant obtenue par lyophilisation d'une émulsion comprenant :

   - une phase discontinue comprenant des gouttelettes incluant un premier et un second composé lipophile, au moins un tensioactif et au moins un médicament; et
- une phase aqueuse continue ;

dans laquelle le premier composé lipophile a une première solubilité massique dans l'eau et le second composé lipophile a une seconde solubilité massique dans l'eau inférieure à la première solubilité massique, dans un ratio volumique du premier composé lipophile au second composé lipophile supérieur à 70 : 30 ; et dans laquelle la phase discontinue est substantiellement exempte ou complètement dépourvue de composé perfluorocarboné.

## Patentansprüche

1. Emulsion zur therapeutischen Verwendung durch kontrollierte Arzneimittelfreisetzung, ausgelöst durch die Anwendung eines externen Stimulus, aufweisend:

   - eine diskontinuierliche Phase, die Tröpfchen aufweist, die eine erste und eine zweite lipophile Verbindung, mindestens ein Tensid und mindestens einen Wirkstoff enthalten; und
   - eine kontinuierliche wässrige Phase;
   wobei die erste lipophile Verbindung eine erste Massenlöslichkeit in Wasser aufweist und die zweite lipophile Verbindung eine zweite Massenlöslichkeit in Wasser, die geringer als die erste Massenlöslichkeit ist, aufweist, in einem Volumenverhältnis der ersten lipophilen Verbindung zur zweiten lipophilen Verbindung von mehr als 70:30; und
   wobei die diskontinuierliche Phase im Wesentlichen frei von oder vollständig frei von Perfluorkohlenstoffverbindungen ist.

2. Emulsion zur therapeutischen Verwendung nach Anspruch 1, wobei die erste Massenlöslichkeit in Wasser bei 25 °C mindestens 10-mal größer ist als die zweite Massenlöslichkeit in Wasser bei 25 °C.

3. Emulsion zur therapeutischen Verwendung nach einem der vorhergehenden Ansprüche, wobei:

   - die erste Massenlöslichkeit in Wasser bei 25 °C mindestens 0,1 $\mu$g/mL beträgt, und/oder
   - die zweite Massenlöslichkeit in Wasser bei 25 °C weniger als 1 $\mu$g/mL beträgt.

4. Emulsion zur therapeutischen Verwendung nach einem der vorhergehenden Ansprüche, wobei die erste und die zweite lipophile Verbindung ein Gemisch bilden, das in einem Gewichtsprozentsatz, bezogen auf das Gesamtgewicht der diskontinuierlichen Phase, von mehr als 60 Gew.-% vorliegt.

5. Emulsion zur therapeutischen Verwendung nach einem der vorhergehenden Ansprüche, wobei die Tröpfchen einen mittleren Durchmesser D zwischen 50 nm und 900 nm aufweisen und der Polydispersitätsindex PDI kleiner als 0,4 ist.

6. Emulsion zur therapeutischen Verwendung nach einem der vorhergehenden Ansprüche, wobei das Volumenverhältnis der ersten lipophilen Verbindung zur zweiten lipophilen Verbindung zwischen 95:5 und 99:1 beträgt.

7. Emulsion für eine therapeutische Verwendung nach einem der vorhergehenden Ansprüche, wobei die zweite lipophile Verbindung mindestens eine lineare Alkylkette mit mindestens 6 Kohlenstoffatomen aufweist.

8. Emulsion für eine therapeutische Verwendung nach einem der Ansprüche 1-6, wobei:

   - die erste lipophile Verbindung ausgewählt ist aus der Gruppe bestehend aus Mono-, Di- oder Tri-Estern von Glycerin oder Glycerinderivaten, Mono-, Di-, Tri- oder Tetra-Estern von Zitronensäuren oder Zitronensäurederivaten, Fettsäuren, Fettsäuremonoestern, Sterolestern, Sphingolipiden, Glycerophospholipiden, Polyketiden, Saccharolipiden, Terpenen, Lipiden, die von Prenol abgeleitet sind, und Kombinationen hiervon; und/oder
   - die zweite lipophile Verbindung ausgewählt ist aus der Gruppe bestehend aus Mono-, Di- oder Tri-Estern von Glycerin oder Glycerinderivaten, Mono-, Di-, Tri- oder Tetra-Estern von Zitronensäuren oder Zitronensäurederivaten, Fettsäuren, Fettsäuremonoestern, Sterolestern, Sphingolipiden, Glycerophospholipiden, Polyketiden, Saccharolipiden, Terpenen, Lipiden, die von Prenol abgeleitet sind, ätherische Ölen, Fettersatzstoffen, Wachsen und Kombinationen davon.

9. Emulsion zur therapeutischen Verwendung nach einem der Ansprüche 1-6, wobei:

- die erste lipophile Verbindung ausgewählt ist aus der Gruppe bestehend aus Acetyltributylcitrat, Propylenglycolmono- oder -dicaprylat und Kombinationen davon; und/oder
- die zweite lipophile Verbindung ist ausgewählt aus der Gruppe bestehend aus C8 bis C10-Triglyceriden, C10 bis C18-Triglyceriden und Kombinationen davon.

10. Emulsion zur therapeutischen Verwendung nach einem der vorhergehenden Ansprüche, wobei die Volumenkonzentration der diskontinuierlichen Phase in der kontinuierlichen wässrigen Phase zwischen 0,000001 % v/v und 90 % v/v beträgt, und/oder wobei die Konzentration des Arzneimittels in Gewicht/Volumen bezogen auf das Gesamtvolumen der Mischung lipophiler Verbindungen in der Emulsion zwischen 10 $\mu$g/mL und 100 mg/mL beträgt.

11. Emulsion zur therapeutischen Verwendung nach einem der vorhergehenden Ansprüche, wobei das mindestens eine Tensid aus der Gruppe ausgewählt ist, die Dendrimere vom Dendri-TAC-Typ, Oligomere vom $F_iTAC_n$- oder $H_iTAC_n$-Typ, TPGS 1000, TPGS 750M, von Zuckern und/oder Aminosäuren abgeleitete Tenside und Kombinationen davon aufweist.

12. Trockenformulierung zur therapeutischen Verwendung durch kontrollierte Freisetzung eines Medikaments, ausgelöst durch die Anwendung eines externen Stimulus, wobei die Trockenformulierung durch Gefriertrocknung einer Emulsion erhalten wird, aufweisend:

- eine diskontinuierliche Phase, die Tröpfchen aufweist, die eine erste und eine zweite lipophile Verbindung, mindestens ein Tensid und mindestens einen Wirkstoff enthalten; und
- eine kontinuierliche wässrige Phase;
wobei die erste lipophile Verbindung eine erste Massenlöslichkeit in Wasser aufweist und die zweite lipophile Verbindung eine zweite Massenlöslichkeit in Wasser, die geringer als die erste Massenlöslichkeit ist, aufweist, in einem Volumenverhältnis der ersten lipophilen Verbindung zur zweiten lipophilen Verbindung von mehr als 70:30; und
wobei die diskontinuierliche Phase im Wesentlichen frei von oder vollständig frei von Perfluorkohlenstoffverbindungen ist.

## Claims

1. An emulsion for therapeutic use by controlled drug release triggered by application of an external stimulus, comprising:

- a discontinuous phase comprising droplets including a first and a second lipophilic compound, at least one surfactant and at least one drug; and
- a continuous aqueous phase;

wherein the first lipophilic compound has a first mass solubility in water and the second lipophilic compound has a second mass solubility in water lower than the first mass solubility, in a volume ratio of the first lipophilic compound to the second lipophilic compound greater than 70: 30; and wherein the discontinuous phase is substantially free or completely free of perfluorocarbon compound.

2. An emulsion for therapeutic use according to claim 1, wherein the first mass solubility in water at 25°C is at least 10 times greater than the second mass solubility in water at 25°C.

3. An emulsion for therapeutic use according to any one of the preceding claims, wherein:

- the first mass solubility in water at 25°C is at least 0.1 $\mu$g/mL, and/or
- the second mass solubility in water at 25°C is less than 1 $\mu$g/mL.

4. An emulsion for therapeutic use according to any one of the preceding claims, wherein the first and second lipophilic compounds form a mixture present in a percentage by weight relative to the total weight of the discontinuous phase of greater than 60% wt.

5. An emulsion for therapeutic use according to any one of the preceding claims, wherein the droplets have an average diameter D of between 50 nm and 900 nm and the polydispersity index PDI is less than 0.4.

6. An emulsion for therapeutic use according to any one of the preceding claims, wherein the volume ratio of the first lipophilic compound to the second lipophilic compound is between 95: 5 and 99: 1.

7. An emulsion for therapeutic use according to any one of the preceding claims, wherein the second lipophilic compound comprises at least one linear alkyl chain of at least 6 carbon atoms.

8. An emulsion for therapeutic use according to any one of claims 1-6, wherein:

   - the first lipophilic compound is selected from the group comprising mono-, di- or tri-esters of glycerol, or derivatives of glycerol, mono-, di-, tri- or tetra-esters of citric acid or derivatives of citric acid, fatty acids, monoesters of fatty acids, sterol esters, sphingolipids, glycerophospholipids, polyketides, saccharolipids, terpenes, prenol-derived lipids, and combinations thereof; and/or
   - the second lipophilic compound is selected from the group comprising mono-, di- or triesters of glycerol, or glycerol derivatives, mono-, di-, tri- or tetraesters of citric acid or citric acid derivatives, fatty acids, fatty acid monoesters, sterol esters, sphingolipids, glycerophospholipids, polyketides, saccharolipids, terpenes, prenol-derived lipids, essential oils, fat substitutes, waxes, and combinations thereof.

9. An emulsion for therapeutic use according to any one of claims 1-6, wherein:

   - the first lipophilic compound is selected from the group comprising acetyltributylcitrate, a propylene glycol mono- or dicaprylate, and combinations thereof; and/or
   - the second lipophilic compound is selected from the group comprising C8 to C10 triglycerides, C10 to C18 triglycerides, and combinations thereof.

10. An emulsion for therapeutic use according to any one of the preceding claims, wherein the concentration by volume of the discontinuous phase in the continuous aqueous phase is between 0.000001% v/v and 90% v/v, and/or wherein the concentration of the drug by weight/volume relative to the total volume of the mixture of lipophilic compounds in the emulsion is between 10 $\mu$g/mL and 100 mg/mL.

11. An emulsion for therapeutic use according to any one of the preceding claims, wherein the at least one surfactant is selected from the group comprising dendrimers of the Dendri-TAC type, oligomers of the $F_iTAC_n$, or $H_iTAC_n$ type, TPGS 1000, TPGS 750M, surfactants derived from sugars and/or amino acids, and combinations thereof.

12. A dry formulation for therapeutic use by controlled release of drug triggered by application of an external stimulus, said dry formulation being obtained by lyophilization of an emulsion comprising:

   - a discontinuous phase comprising droplets including a first and a second lipophilic compound, at least one surfactant and at least one drug; and
   - a continuous aqueous phase;

   wherein the first lipophilic compound has a first mass solubility in water and the second lipophilic compound has a second mass solubility in water lower than the first mass solubility, in a volume ratio of the first lipophilic compound to the second lipophilic compound greater than 70: 30; and wherein the discontinuous phase is substantially free or completely free of perfluorocarbon compound.

FIG.1

FIG.2

FIG.3

**EP 4 157 222 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3095806 A1

- WO 03000295 A2

**Littérature non-brevet citée dans la description**

- **T. WOOSTER et al.** Impact of Oil Type on Nanoemulsion Formation and Ostwald Ripening Stability. *Langmuir,* 2008, vol. 24, 12758-12765

- **CONTINO-PEPIN et al.** Amphiphilic Oligomers: A New Kind of Macromolecular Carrier of Antimitotic Drugs. *Curr. Med. Chem. - Anti-Cancer Agents,* 2002, vol. 2, 645-665